(19) Europäisches Patentamt
European Patent Office
Office européen des brevets

(11) **EP 3 741 738 B1**

(12) **EUROPÄISCHE PATENTSCHRIFT**

(45) Veröffentlichungstag und Bekanntmachung des Hinweises auf die Patenterteilung:
**21.09.2022  Patentblatt 2022/38**

(21) Anmeldenummer: **19020337.2**

(22) Anmeldetag: **22.05.2019**

(51) Internationale Patentklassifikation (IPC):
**C07C 29/151** (2006.01)     **C07C 31/04** (2006.01)

(52) Gemeinsame Patentklassifikation (CPC):
(C-Sets verfügbar)
**C07C 29/1512**                    (Forts.)

(54) **VERFAHREN ZUR HERSTELLUNG VON METHANOL**

PROCESS FOR MANUFACTURING METHANOL

PROCÉDÉ DE FABRICATION DE MÉTHANOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**25.11.2020   Patentblatt 2020/48**

(73) Patentinhaber: **L'AIR LIQUIDE, SOCIÉTÉ ANONYME POUR L'ÉTUDE ET L'EXPLOITATION DES PROCÉDÉS GEORGES CLAUDE**
**75007 Paris (FR)**

(72) Erfinder:
• **Castillo-Welter, Frank**
  **61381 Friedrichsdorf (DE)**
• **Haag, Stephane**
  **60598 Frankfurt am Main (DE)**
• **Schuhmann, Timm**
  **63067 Offenbach (DE)**
• **Frind, Robert**
  **01239 Dresden (DE)**

(74) Vertreter: **Stang, Stefan**
**Air Liquide Forschung und Entwicklung GmbH**
**Gwinnerstraße 27-33**
**60388 Frankfurt am Main (DE)**

(56) Entgegenhaltungen:
**EP-A1- 3 205 622     WO-A1-2017/121981**
**GB-A- 2 550 993**

(52) Gemeinsame Patentklassifikation (CPC): (Forts.)

C-Sets
**C07C 29/1512, C07C 31/04**

C-Sets
**C07C 29/1512, C07C 31/04**

**Beschreibung**

**Gebiet der Erfindung**

Die Erfindung betrifft ein Verfahren zur Herstellung von Methanol aus einem Kohlenmonoxid (CO) und Wasserstoff ($H_2$) aufweisenden Einsatzgas. Die Erfindung betrifft ferner die Verwendung einer Anlage zur Herstellung von Methanol aus einem Kohlenmonoxid (CO) und Wasserstoff ($H_2$) aufweisenden Einsatzgas.

**Stand der Technik**

Methanol wird heute üblicherweise aus Synthesegas, einem vorwiegend Kohlenmonoxid (CO) und Wasserstoff ($H_2$), sowie in der Regel auch Kohlendioxid ($CO_2$) aufweisenden Einsatzgas hergestellt. In den modernen sogenannten Niederdruckverfahren werden dabei Drücke zwischen 60 und 120 bar bei der katalytischen Umsetzung des Synthesegases zu Rohmethanol verwendet. Die Reaktion findet dabei an Festbett-Katalysatoren bei moderaten Temperaturen von unter 300 °C statt. Als Katalysatoren kommen beispielsweise Materialien auf Kupfer-Zinkoxid-Aluminiumoxid Basis zum Einsatz.

Synthesegas aus Dampfreformierung wird meist bei Drücken zwischen 20 und 40 bar erzeugt und muss zunächst über einen Synthesegas-Kompressor auf den für die Methanolsynthese erforderlichen höheren Druck (Reaktionsdruck) von mindestens 60 bar verdichtet werden. Bekannte Verfahren arbeiten dabei mit hohen Gaskreisläufen in einem sogenannten Synthesekreislauf, in dem die Menge des im Kreislauf geführten Gases bis zu fünfmal höher ist als die zugeführte Frischgasmenge aus der Synthesegaserzeugung. Alternativ zu einem Synthesekreislauf kann die Erzeugung des Rohmethanols auch in einem mehrstufigen Reaktorsystem mit seriell angeordneten Reaktoren erfolgen. Die Anzahl der Reaktoren richtet sich dabei nach dem pro Reaktor in einem Durchgang (*per pass*) erzielbaren Umsatz.

Die für die Verdichtung des Synthesegases erforderlichen Gaskompressoren sind Maschinen, welche einerseits aufgrund ihres hohen Energieverbrauchs hohe Betriebskosten (OPEX) verursachen, und andererseits aufgrund ihrer Größe und Konstruktionsweise hohe Investitionskosten erfordern (CAPEX). Die für die Verdichtung der Einsatzgase, beispielsweise Synthesegas, verwendeten Gaskompressoren können inklusive Antriebstechnik bis zu 30 % der Kosten aller Prozesshauptausrüstungen betragen. Somit geht die für die Verdichtung von Gasen erforderliche Kompressorleistung stark in die Gesamtkosten einer Anlage zur Methanolherstellung ein, da mit der Menge der zu komprimierenden Gase nicht nur die die Größe der Kompressoren steigt, sondern auch die für den Betrieb dieser Kompressoren erforderliche Leistung.

Der Hauptreaktorstufe einer Methanol-Synthese, welche den Synthesekreislauf oder das mehrstufige Reaktorsystem umfasst, kann eine Vorreaktorstufe vorgeschaltet sein, in der ein Teil des Synthesegases vor Einschleusen in die Hauptreaktorstufe zu Methanol umgesetzt wird. Die Vorreaktorstufe weist üblicherweise einen einzelnen Vorreaktor auf, den das Synthesegas in einem einzigen Durchgang passiert, und an dessen Ausgang das erzeugte Methanol abgeschieden (kondensiert) wird, bevor das verbleibende Synthesegas (Restgas) zur Hauptreaktorstufe weitergeführt wird. Die Verfahrensführung mit einem Vorreaktor hat den Vorteil, dass nicht die Gesamtmenge des Synthesegases vom Synthesegaskompressor auf den Druck in der Hauptreaktorstufe verdichtet werden muss, sondern nur noch das verbleibende Synthesegas (Restgas).

Im Vorreaktor kann das aus der Synthesegaserzeugung zugeführte Einsatzgas ohne vorherige Verdichtung mit einem Kompressor zu Methanol umgesetzt werden, wie beispielsweise in der DE 101 26 719 A1 beschrieben. Die dabei erzielbaren Energieeinsparungen für den dem Vorreaktor nachgeschalteten Synthesegaskompressor sind dabei häufig gering, da in der Vorreaktorstufe nur ein geringer Umsatz von Synthesegas zu Methanol erzielt wird. Dies führt letztlich dazu, dass trotz Verwendung eines Vorreaktors, welcher die Investitions- und Betriebskosten der Anlage erhöht, die Hauptmenge des Synthesegases in die Hauptreaktorstufe einzuschleusen ist. Die in Bezug auf den der Vorreaktorstufe nachgeschalteten Synthesegaskompressor eingesparten Kosten (OPEX und CAPEX) werden somit durch die Verwendung des Vorreaktors im negativen Sinne kompensiert.

GB 2550993 beschreibt ein in einem Kreislauf geführtes Verfahren zur Methanolsynthese, welches zwei Synthesereaktoren umfasst. Beide Reaktoren enthalten einen gekühlten Methanolsynthese-Katalysator, wobei der erste Synthesereaktor eine höhere Wärmeübertragung pro Kubikmeter Katalysator aufweist als der zweite Synthesereaktor. Die Menge des zu den Reaktoren zurückgeführten Kreislaufgases unterscheidet sich dabei jeweils im Rückführungsverhältnis zum eingesetzten Frischgas. Ein ähnliches Verfahren wird durch die WO 2017/121981 offenbart.

**Beschreibung der Erfindung**

Die Aufgabe der vorliegenden Erfindung liegt somit darin, die Nachteile des Standes der Technik zumindest teilweise zu überwinden.

Eine weitere Aufgabe der vorliegenden Erfindung liegt darin, ein Verfahren bereitzustellen, das signifikant höhere Energieeinsparungen in Bezug auf die für die Verdichtung von Einsatzgasen verwendeten Gaskompressoren ermöglicht.

Eine weitere Aufgabe der vorliegenden Erfindung liegt darin, ein Verfahren bereitzustellen, welches den Umsatz an Einsatzgas, insbesondere Synthesegas, im Vorreaktor erhöht, so dass die anschließend zu verdichtende Restgasmenge signifikant verringert wird.

Die Aufgaben der Erfindung werden zumindest teilweise gelöst durch ein Verfahren zur Herstellung von Methanol aus einem Kohlenmonoxid (CO) und Wasserstoff (H$_2$) aufweisenden Einsatzgas, bei dem unter erhöhtem Druck erzeugtes und stehendes Einsatzgas als Einsatzgasstrom zur katalytischen Umsetzung in einen ersten methanolhaltigen Produktstrom in eine Vorreaktorstufe eingeschleust wird, und Methanol aus dem ersten methanolhaltigen Produktstrom abgetrennt und aus der Vorreaktorstufe ausgeschleust wird, und Restgas des verbleibenden Einsatzgasstroms als Restgasstrom auf Reaktionsdruck verdichtet und zur katalytischen Umsetzung in einen zweiten methanolhaltigen Produktstrom in eine Hauptreaktorstufe eingeschleust wird, und Methanol aus dem zweiten methanolhaltigen Produktstrom abgetrennt und aus der Hauptreaktorstufe ausgeschleust wird. Erfindungsgemäße ist vorgesehen, dass das Einsatzgas vor dem Einschleusen in die Vorreaktorstufe einen Kohlenmonoxid-Gehalt von 25 Vol.-% bis 36 Vol.-% aufweist.

Bevorzugt weist das Einsatzgas vor Einschleusen in die Vorreaktorstufe einen Kohlenmonoxid-Gehalt von 27 Vol.-% bis 33 Vol.-% auf, besonders bevorzugt einen Kohlenmonoxid-Gehalt von 28 Vol.-% bis 31 Vol.-%, und weiter bevorzugt einen Kohlenmonoxid-Gehalt von 29 Vol.-% bis 30 Vol.-%.

Vorzugsweise handelt es sich bei dem Einsatzgas um ein Synthesegas aus Kohlevergasung.

Es wurde überraschend festgestellt, dass die Energieeinsparungen im dem Vorreaktor nachgeschalteten Gaskompressor bei gleichzeitiger Verwendung eines Vorreaktors bei einem Einsatzgas mit einem erfindungsgemäßen Kohlenmonoxid-Gehalt, beispielsweise einem Einsatzgas aus Kohlevergasung, deutlich höher sind als dies bei einem Einsatzgas mit niedrigerem Kohlenmonoxid-Gehalt der Fall ist. Einsatzgase mit einem niedrigeren Kohlenmonoxid-Gehalt sind beispielsweise Einsatzgase aus Dampfreformierung von Methan (*steam methane reforming - SMR*), oder einer Kombination von Dampfreformierung und autothermer Reformierung (sogenanntes *combined reforming*). Auf Basis dieser Technologien erzeugte Synthesegase weisen regelmäßig CO-Gehalte von deutlich unter 25 Vol.-% auf.

Aufgrund des höheren CO-Gehalts im dem Vorreaktor zugeführten Einsatzgas, beispielsweise einem Einsatzgas aus Kohlevergasung, kann sowohl die Kompressorleistung (Energieverbrauch des Kompressors) als auch die Kompressorgröße eines Einsatzgaskompressors zur Verdichtung des Restgasstroms, insbesondere eines Synthesegaskompressors, überraschend deutlich verringert werden. Dadurch ergeben sich signifikante Einsparungen in Bezug auf OPEX und CAPEX des Einsatzgaskompressors.

Eine bevorzugte Ausführungsform des erfindungsgemäßen Verfahrens ist dadurch gekennzeichnet, dass das Einsatzgas vor dem Einschleusen in die Vorreaktorstufe einen Wasserstoff-Gehalt von 66 Vol.-% bis 72 Vol.-% aufweist.

Besonders bevorzugt weist das Einsatzgas vor dem Einschleusen in die Vorreaktorstufe einen Wasserstoff-Gehalt von 67 Vol.-% bis 70 Vol.-% auf, und weiter bevorzugt einen Wasserstoff-Gehalt von 68 Vol.-% bis 69 Vol.-%.

Untersuchungen haben gezeigt, dass in Kombination mit den erfindungsgemäßen Kohlenmonoxid-Gehalten im Einsatzgas besonders hohe Ausbeuten an Methanol im Vorreaktor erzielt werden, so dass sich weitere Einsparungen in Bezug auf den Einsatzgaskompressor ergeben.

Eine bevorzugte Ausführungsform des erfindungsgemäßen Verfahrens ist dadurch gekennzeichnet, dass das Einsatzgas vor dem Einschleusen in die Vorreaktorstufe einen Kohlendioxid-Gehalt (CO$_2$ - Gehalt) von kleiner oder gleich 5 Vol.-% aufweist.

Vorzugsweise weist das Einsatzgas vor dem Einschleusen in die Vorreaktorstufe einen Kohlendioxid-Gehalt von 2 Vol.-% bis 4 Vol.-% auf, besonders bevorzugt von 2,5 Vol.-% bis 3,5 Vol.-%.

Untersuchungen haben gezeigt, dass in Kombination mit den erfindungsgemäßen Kohlenmonoxid-Gehalten und optional Wasserstoff-Gehalten im Einsatzgas besonders hohe Ausbeuten an Methanol im Vorreaktor erzielt werden, so dass sich weitere Einsparungen in Bezug auf den Einsatzgaskompressor ergeben. Insbesondere wurde gefunden, dass die erfindungsgemäßen Kohlendioxid-Gehalte zu einer besonders schnellen Einstellung des thermodynamischen Gleichgewichts der Methanolsynthesereaktion führen, und somit kinetisch bevorzugt sind. Auf Basis von SMR, oder einer Kombination aus autothermer Reformierung und SMR erzeugte Synthesegase weisen regelmäßig CO$_2$-Gehalte von deutlich über 5 Vol.-% auf und sind daher weniger bevorzugt.

Eine bevorzugte Ausführungsform des erfindungsgemäßen Verfahrens ist dadurch gekennzeichnet, dass das Einsatzgas vor dem Einschleusen in die Vorreaktorstufe eine Stöchiometriezahl SN, wobei gilt

$$SN = \frac{n(H_2) - n(CO_2)}{n(CO) + n(CO_2)}$$

, *mit n in [mol]*,
von 1,5 bis 3,0 aufweist.

Die Stöchiometriezahl gibt die stöchiometrischen Verhältnisse der Methanolsynthesereaktion

$$CO + 2\,H_2 \rightleftharpoons CH_3OH$$

wieder. Bei idealen stöchiometrischen Verhältnissen und der Abwesenheit von Kohlendioxid liegt die Stöchiometriezahl bei 2. Untersuchungen haben jedoch gezeigt, dass kleinere Kohlendioxid-Mengen den CO-Umsatz im Vorreaktor erhöhen können, so dass die Stöchiometriezahl vorzugsweise in einem Bereich von 1,8 bis 3,0 variiert wird. Bevorzugt gilt dabei $1,8 \leq SN \leq 2,5$ und besonders bevorzugt $1,9 \leq SN \leq 2,2$ und weiter bevorzugt $1,95 \leq SN \leq 2,05$.

Eine bevorzugte Ausführungsform des erfindungsgemäßen Verfahrens ist dadurch gekennzeichnet, dass das Einsatzgas einen Druck von 20 bis 100 bar aufweist, vorzugsweise einen Druck von 35 bis 55 bar aufweist.

Untersuchungen haben gezeigt, dass im erfindungsgemäßen Druckbereich in Kombination mit den erfindungsgemäßen Kohlenmonoxid-Gehalten, optional Wasserstoff-Gehalten, sowie optional Kohlendioxid-Gehalten im Einsatzgas besonders hohe Ausbeuten an Methanol im Vorreaktor erzielt werden, so dass sich weitere Einsparungen in Bezug auf den Einsatzgaskompressor ergeben.

Eine bevorzugte Ausführungsform des erfindungsgemäßen Verfahrens ist dadurch gekennzeichnet, dass das unter erhöhtem Druck erzeugte und stehende Einsatzgas ohne weitere Druckerhöhung in die Vorreaktorstufe eingeschleust wird. Insbesondere wird das unter erhöhtem Druck erzeugte und stehende Einsatzgas ohne weitere Druckerhöhung durch einen Gaskompressor in die Vorreaktorstufe eingeschleust. Das Wasserstoff und Kohlenmonoxid aufweisende Einsatzgas wird üblicherweise bei erhöhtem Druck, beispielsweise im Rahmen einer Kohlevergasung, erzeugt und kann ohne weitere Verdichtung, beispielsweise durch einen Gaskompressor, in den Vorreaktor eingeschleust werden. Dadurch reduziert sich die Gesamtzahl erforderlicher Kompressoren, beispielsweise auf einen Gaskompressor zur Verdichtung des Restgases aus der Vorreaktorstufe sowie einen Gaskompressor zur Verdichtung des Rückführgases (Recycle-Gases) bei Verwendung eines Synthesekreislaufs.

Eine bevorzugte Ausführungsform des erfindungsgemäßen Verfahrens ist dadurch gekennzeichnet, dass das Restgas des Restgasstroms vor dem Einschleusen in die Hauptreaktorstufe auf einen gegenüber dem Einsatzgas höheren Druck verdichtet wird. Vorzugsweise wird der Restgasstrom vor dem Einschleusen in die Hauptreaktorstufe durch einen Gaskompressor auf einen höheren Druck verdichtet.

Selbst bei den sogenannten Niederdruck-Verfahren zur Herstellung von Methanol sind in der Hauptreaktorstufe Drücke von 60 bar bis 120 bar erforderlich, auf welche das Restgas des Restgasstroms vor dem Einschleusen in die Hauptreaktorstufe zu verdichten ist. Der Druck in der Hauptreaktorstufe ist regelmäßig höher als der Druck des unter erhöhtem Druck erzeugten und stehenden Einsatzgases.

Dabei wird der Restgasstrom vor dem Einschleusen in die Hauptreaktorstufe wenigstens auf einen um zumindest 5 bar höheren Druck verdichtet als es dem Druck des Einsatzgases entspricht, oder um einen um zumindest 10 bar höheren Druck, oder um einen zumindest 25 bar höheren Druck, oder um einen zumindest 40 bar höheren Druck.

Eine bevorzugte Ausführungsform des erfindungsgemäßen Verfahrens ist dadurch gekennzeichnet, dass die Hauptreaktorstufe Teil eines Synthesekreislaufs ist, wobei ein nach Abtrennung des Methanols aus dem zweiten methanolhaltigen Produktstrom verbleibender Rückführgasstrom zur Hauptreaktorstufe im Synthesekreislauf zurückgeführt und mit dem Restgasstrom zusammengeführt wird. Dabei wird auch das Rückführgas des Rückführgasstroms auf einen gegenüber dem Einsatzgas erhöhten Druck verdichtet, bevor oder wobei das Rückführgas (Recycle-Gas) mit dem Restgas zusammengeführt wird. Die Verdichtung geschieht ebenfalls mit Hilfe eines Gaskompressors, in diesem Fall als Rückführgaskompressor oder Recycle-Gas-Kompressor bezeichnet. Alternativ dazu ist die Verwendung eines einzigen insbesondere mehrstufigen Gaskompressors denkbar, der den Restgasstrom und den Rückführgasstrom auf den für die Methanolsynthese in der Hauptreaktorstufe erforderlichen Reaktionsdruck verdichtet. Nach Verdichtung auf Reaktionsdruck werden Rückführgas und Restgas als kombinierter Gasstrom in der Hauptreaktorstufe zu einem zweiten methanolhaltigen Produktstrom umgesetzt.

Eine bevorzugte Ausführungsform des erfindungsgemäßen Verfahrens mit Synthesekreislauf ist dadurch gekennzeichnet, dass für eine Rezirkulationsrate R, definiert als

$$R = \frac{Volumenstrom\ (R\ddot{u}ckf\ddot{u}hrgasstrom)}{Volumenstrom\ (Restgasstrom)},$$

$R \leq 2,5$ gilt.

Dabei gilt vorzugsweise $1,5 \leq R \leq 2,5$ und gilt besonders bevorzugt $1,5 \leq R \leq 2$.

Eine bevorzugte Ausführungsform des erfindungsgemäßen Verfahrens ist dadurch gekennzeichnet, dass die Hauptreaktorstufe eine Anzahl n seriell angeordneter Teilreaktorstufen umfasst, und der zweite methanolhaltige Produktstrom n methanolhaltige Teilproduktströme umfasst, wobei Methanol aus den n methanolhaltigen Teilproduktströmen abgetrennt und aus der Hauptreaktorstufe ausgeschleust wird.

Alternativ zur Ausgestaltung der Hauptreaktorstufe als Synthesekreislauf ist die Ausgestaltung der Hauptreaktorstufe als System seriell angeordneter Teilreaktorstufen denkbar. Vorzugsweise entspricht dabei eine Teilreaktorstufe einem Reaktor, in den ein n-ter Teilrestgasstrom eingeschleust wird, wobei die Teilreaktorstufe vom n-ten Teilrestgasstrom einmalig, in einem Durchgang (*single-pass*) durchströmt wird, und aus der Teilreaktorstufe jeweils ein n-ter methanolhaltiger Teilproduktstrom ausgeschleust wird.

Eine bevorzugte Ausführungsform des erfindungsgemäßen Verfahrens ist dadurch gekennzeichnet, dass die Vorreaktorstufe einen wassergekühlten Reaktor umfasst. Die Kühlung des Reaktors der Vorreaktorstufe erfolgt vorzugsweise durch unter erhöhtem Druck siedendes Wasser, wobei der dabei erzeugte Wasserdampf einer Dampftrommel zur Energiegewinnung zugeführt wird.

Eine bevorzugte Ausführungsform des erfindungsgemäßen Verfahrens ist dadurch gekennzeichnet, dass der wassergekühlte Reaktor eine Kühltemperatur von 180 °C bis 250 °C aufweist. Vorzugsweise weist der wassergekühlte Reaktor eine Kühltemperatur von kleiner als 235 °C auf, insbesondere und vorzugsweise eine Kühltemperatur von 200 °C bis 235 °C.

Dabei wird unter der Kühltemperatur die Temperatur des zur Kühlung verwendeten Mediums am Ausgang der gekühlten Seite des Reaktors verstanden. Auf der Prozessseite gleicht sich die Temperatur entlang des Katalysatorbetts an die gewählte Kühltemperatur an. Dabei darf eine bestimmte Temperatur nicht unterschritten werden, um sicher oberhalb der Zündtemperatur des Katalysators zu arbeiten.

Eine bevorzugte Ausführungsform des erfindungsgemäßen Verfahrens ist dadurch gekennzeichnet, dass die Hauptreaktorstufe einen wassergekühlten Reaktor umfasst. Vorzugsweise umfasst die Hauptreaktorstufe zusätzlich einen gasgekühlten Reaktor, wobei der gasgekühlte Reaktor insbesondere stromabwärts des wassergekühlten Reaktors angeordnet ist.

Bei Verwendung einer Kombination aus wassergekühltem und gasgekühltem Reaktor fallen die Einsparungen bezüglich der Kompressorleistung besonders hoch aus, wie Untersuchungen gezeigt haben. Dafür sind die Aufwendungen bezüglich des insgesamt erforderlichen Katalysatorvolumens aufgrund der Verwendung von zwei Reaktoren etwas höher, was jedoch durch die zusätzlich eingesparte Kompressorleistung kompensiert wird.

Eine dazu alternative Ausführungsform ist dadurch gekennzeichnet, dass die Hauptreaktorstufe keinen gasgekühlten Reaktor umfasst. Insbesondere umfasst die Hauptreaktorstufe ausschließlich einen wassergekühlten Reaktor.

Wie Untersuchungen gezeigt haben, sind die Einsparungen bezüglich der Kompressorleistung bei der Verwendung eines einzigen wassergekühlten Reaktors nur unwesentlich niedriger im Vergleich zu einer Konfiguration, welche einen wassergekühlten und einen gasgekühlten Reaktor umfasst. Die etwas höhere erforderliche Kompressorleistung wird jedoch dadurch kompensiert, dass das gesamte Katalysatorvolumen reduziert werden kann.

Weisen sowohl die Vorreaktorstufe als auch die Hauptreaktorstufe jeweils einen wassergekühlten Reaktor auf, können beide wassergekühlten Stufen mit demselben Kühlsystem betrieben werden. Mit anderen Worten, die Vorreaktorstufe und die Hauptreaktorstufe werden durch ein gemeinsames Kühlsystem gespeist. Vorzugsweise weisen beide Stufen die gleiche soll-Kühltemperatur auf.

Vorgenannte Konfigurationen kommen insbesondere dann zum Einsatz, wenn die Hauptreaktorstufe als Synthesekreislauf ausgestaltet ist. Der Synthesekreislauf weist dann vorzugsweise entweder einen wassergekühlten Reaktor und stromabwärts einen gasgekühlten Reaktor auf, oder alternativ dazu ausschließlich einen wassergekühlten Reaktor.

Eine Anlage zur Herstellung von Methanol aus einem Kohlenmonoxid (CO) und Wasserstoff ($H_2$) aufweisenden Einsatzgas weist folgende Komponenten auf. Eine Zuführleitung zum Einschleusen von unter erhöhtem Druck stehendem Einsatzgas in eine Vorreaktorstufe, wobei die Vorreaktorstufe zur katalytischen Umsetzung des Einsatzgases in einen ersten methanolhaltigen Produktstrom ausgelegt ist;

einen stromabwärts der Vorreaktorstufe angeordneten und über eine Leitung mit der Vorreaktorstufe verbundenen ersten Abscheider zur Abtrennung von Methanol aus dem ersten methanolhaltigen Produktstrom und zur Abtrennung eines Restgasstroms;

eine mit dem ersten Abscheider verbundene Zuführleitung zum Einschleusen des Restgasstroms in einen Gaskompressor;

eine Zuführleitung zum Einschleusen des im Gaskompressor auf Reaktionsdruck verdichteten Restgasstroms in eine Hauptreaktorstufe, wobei die Hauptreaktorstufe zur katalytischen Umsetzung des Restgasstroms in einen zweiten methanolhaltigen Produktstrom ausgelegt ist;

einen stromabwärts der Hauptreaktorstufe angeordneten und über eine Leitung mit der Hauptreaktorstufe verbundenen zweiten Abscheider zur Abtrennung von Methanol aus dem zweiten methanolhaltigen Produktstrom. Die Anlage ist dadurch gekennzeichnet, dass die Vorreaktorstufe zur Umsetzung eines Einsatzgases mit einem Kohlenmonoxid-Gehalt von 25 Vol.-% bis 36 Vol.-% ausgelegt ist.

Die Aufgaben der Erfindung werden ferner zumindest teilweise gelöst durch Verwendung einer Anlage, insbesondere der vorgenannten Anlage, zur Herstellung von Methanol aus einem Kohlenmonoxid (CO) und Wasserstoff (H2) aufweisenden Einsatzgas in einem erfindungsgemäßen Verfahren zur Umsetzung eines Einsatzgases mit einem Kohlenmonoxid-Gehalt von 25 Vol.-% bis 36 Vol.-%.

Dabei ist bevorzugt, dass das Einsatzgas mittels Vergasung von Kohle, durch autothermes Reformieren oder Gas-POX erzeugt wurde.

Einsatzgas

Das Einsatzgas ist ein Gasgemisch, welches zumindest Kohlenmonoxid (CO) und Wasserstoff ($H_2$) als Gaskomponenten aufweist. Dadurch ist das Einsatzgas zur Herstellung von Methanol geeignet. Insbesondere handelt es sich bei dem Einsatzgas, je nach Art der Herstellung, um ein Synthesegas, Wassergas oder Spaltgas. Vorzugsweise, jedoch ohne darauf beschränkt zu sein, wird das Einsatzgas durch Vergasung von Kohle erzeugt. Auch reine Autotherm-Reformer (ATR) oder Anlagen für die partielle Oxidation von gasförmigen kohlenstoffhaltigen Edukten (Gas-POX) können Synthesegase mit hohem CO-Gehalt erzeugen, welche für das erfindungsgemäße Verfahren geeignet sind.

Als Kohlenmonoxid-Quelle kommt ferner eine Elektrolyse von Kohlendioxid in Frage, die vorzugsweise unter Nutzung von Strom aus erneuerbaren Energiequellen einen CO-reichen Stoffstrom erzeugt.

Das Einsatzgas gemäß einer Ausführungsform der Erfindung weist einen Kohlenmonoxid-Gehalt von 25 Vol.-% bis 36 Vol.-% auf. Die Gehaltsangabe sowie weitere in Zusammenhang mit der Erfindung erwähnte Gehaltsangaben beziehen sich dabei stets auf das trockene Einsatzgas. Trockenes Einsatzgas bedeutet, dass Wasser vollständig oder im Wesentlichen vollständig aus dem Einsatzgas entfernt wurde. In einem Beispiel wurde Wasser bis zu einem Restgehalt von nicht mehr als 1 Vol.-% entfernt, oder nicht mehr als 0,5 Vol.-%, oder nicht mehr als 0,3 Vol.-%, oder nicht mehr als 0,1 Vol.-%.

Üblicherweise weist das Einsatzgas Kohlendioxid ($CO_2$) als weitere Komponente auf. In bestimmten Fällen wird Kohlendioxid im Rahmen einer Gaswäsche vollständig, beispielsweise einer Gaswäsche mit Methanol als Waschmittel, vollständig oder zumindest bis in den Spurenbereich entfernt.

Optionale Komponenten im Einsatzgas sind beispielsweise Methan ($CH_4$) und inerte Bestandteile wie Stickstoff ($N_2$) oder Argon (Ar).

Das Einsatzgas, insbesondere Einsatzgas aus einer Kohlevergasung, wird üblicherweise vor seiner Verwendung einer Gaswäsche zur Entfernung von schwefelhaltigen Komponenten wie Schwefelwasserstoff ($H_2S$) sowie Carbonylsulfid (COS) unterzogen, da schwefelhaltige Substanzen Gifte für die bei der Methanolsynthese verwendeten Katalysatoren darstellen. Im Rahmen der Gaswäsche wird üblicherweise auch Kohlendioxid bis zu einem gewissen Ausmaß entfernt, jedoch erfolgt die Kohlendioxid-Entfernung in den meisten Fällen nicht vollständig. Bei der Gaswäsche kann es sich beispielsweise um ein Verfahren nach dem Prinzip der physikalischen oder chemischen Absorption handeln. Ein Beispiel für ein Gaswäscheverfahren nach dem Prinzip der physikalischen Absorption ist die Methanolwäsche, auch als Rectisol-Verfahren bekannt. Ein Beispiel für ein Gaswäscheverfahren nach dem Prinzip der chemischen Absorption ist die Gaswäsche mit Hilfe von Aminen.

Zur Einstellung eines bestimmten Verhältnisses von Kohlenmonoxid zu Wasserstoff, und/oder zur Einstellung eines bestimmten Anteils von Kohlendioxid im Einsatzgas kann das Einsatzgas teilweise Synthesegas aus einer Wassergas-Shift-Reaktion enthalten. Die Wassergas-Shift-Reaktion dient der Erhöhung des Wasserstoffanteils und gleichzeitigen Verringerung des Kohlenmonoxid-Anteils in einem Synthesegas, Wassergas oder Spaltgas. Da das Einsatzgas vorzugsweise einen hohen Kohlenmonoxid-Gehalt aufweisen soll, ist der Anteil an einer Wassergas-Shift-Reaktion unterzogenem Gas im Einsatzgas vorzugsweise gering.

Das Einsatzgas wird unter erhöhtem Druck erzeugt und steht vor Einschleusen in die Vorreaktorstufe unter erhöhtem Druck. Üblicherweise erfolgt die Herstellung des Einsatzgases bei einem Druck, der deutlich oberhalb des Umgebungsdrucks liegt, regelmäßig bei wenigstens 20 bar und bis zu 100 bar. Der Druck, bei welchem das Einsatzgas erzeugt wird entspricht im Wesentlichen dem Druck, unter dem das Einsatzgas vor Einschleusen in die Vorreaktorstufe steht. Aufgrund nicht zu vermeidender Druckverluste ist der Druck bei Einschleusen in den Vorreaktor etwas niedriger als bei der Erzeugung.

Vorreaktorstufe, Hauptreaktorstufe, Restgasstrom

Das Einsatzgas wird zunächst der Vorreaktorstufe zugeführt. Dort findet eine teilweise Umsetzung des Einsatzgases zu einem ersten methanolhaltigen Produktstrom statt, welcher neben Methanol und, in Abhängigkeit vom Kohlendioxid-Gehalt des Einsatzgases, Wasser enthält. Ferner weist der erste methanolhaltige Produktstrom (sowie weitere methanolhaltige Produktströme) Nebenprodukte wie beispielsweise Dimethylether, Methylformiat, Aceton, Ethanol sowie höhere Alkohole auf. Das Gemisch des methanolhaltigen Produktstroms wird vorzugsweise nach Kondensation in einem Abscheider aus der Vorreaktorstufe ausgeschleust. Verbleibendes, nicht in der Vorreaktorstufe umgesetztes Einsatzgas - auch als Restgas bezeichnet - wird als Restgasstrom der Hauptreaktorstufe zugeführt. In der Hauptreaktorstufe findet die weitgehend vollständige Umsetzung des Restgases zu einem wiederum Methanol, Wasser und Nebenprodukte aufweisenden (zweiten) methanolhaltigen Produktstrom statt. Der zweite methanolhaltige Produktstrom wird wiederum vorzugsweise nach Kondensation in einem Abscheider aus der Hauptreaktorstufe ausgeschleust.

Der Druck in der Hauptreaktorstufe ist grundsätzlich höher als der Druck in der Vorreaktorstufe, so dass ein Gaskompressor - hier auch als Restgaskompressor bezeichnet - erforderlich ist, um das Restgas auf den in der Hauptreaktorstufe erforderlichen Druck zu verdichten.

Methanolhaltige Produkströme aus Vorreaktorstufe und Hauptreaktorstufe werden üblicherweise zusammengeführt und aufgearbeitet, um Methanol mit einem vorgegebenen Reinheitsgrad zu erzeugen.

Reaktionsdruck

Reaktionsdruck ist der für die katalytische Reaktion der Bestandteile des Restgases und/oder Rückführgases zu Methanol vorherrschende und erforderliche Druck, mit dem Restgas und/oder Rückführgas in die Hauptreaktorstufe eingeschleust wird.

In einem Beispiel beträgt der Reaktionsdruck im betreffenden Reaktor 60 bis 120 bar, vorzugsweise 70 bis 100 bar, besonders bevorzugt 75 bis 90 bar und weiter bevorzugt 75 bis 85 bar.

Synthesekreislauf und Rückführgasstrom

Ist die Hauptreaktorstufe als Synthesekreislauf ausgestaltet, wird dem Reaktor oder den (seriell angeordneten) Reaktoren der Hauptreaktorstufe ein Teil Restgas als Restgasstrom aus der Vorreaktorstufe zugeführt, und ein Teil Rückführgas (Recycle-Gas) zugeführt, welches den Reaktor oder die Reaktoren der Hauptreaktorstufe bereits passiert hat. Sowohl der Restgasstrom als auch der Rückführgasstrom werden in einem Beispiel jeweils durch einen dedizierten Gaskompressor auf den für die Methanolsynthesereaktion erforderlichen Druck der Hauptreaktorstufe verdichtet. In einem weiteren Beispiel wird ein einziger Gaskompressor zur Kompression beider Gasströme verwendet, der über mehrere Kompressionsstufen verfügt. Der Rückführgasstrom kann in diesem Fall zusammen mit dem Restgasstrom direkt der zweiten Kompressionsstufe zugeführt werden, wobei beide Gasströme auf Reaktionsdruck verdichtet werden.

Das Verhältnis des Volumenstroms des Rückführgasstroms zum Volumenstrom des Restgasstroms wird als Rezirkulationsrate R bezeichnet.

Teilreaktorstufe

Umfasst die Vorreaktorstufe oder Hauptreaktorstufe jeweils mehrere Reaktoren, so kann jeder der Reaktoren auch als Teilreaktorstufe (der Vorreaktorstufe oder Hauptreaktorstufe) bezeichnet werden.

**Ausführungsbeispiele**

Die Erfindung wird im Folgenden durch Beispiele näher erläutert, ohne den Gegenstand der Erfindung dadurch zu beschränken. Weitere Merkmale, Vorteile und Anwendungsmöglichkeiten der Erfindung ergeben sich aus der nachfolgenden Beschreibung der Ausführungsbeispiele in Zusammenhang mit den Zeichnungen und den Zahlenbeispielen.

Es zeigt

Figur 1      eine schematische Fließbilddarstellung eines erfindungsgemäßen Verfahrens 100 oder einer erfindungsgemäßen Anlage 100 gemäß einem ersten Beispiel der Erfindung und

Figur 2      eine schematische Fließbilddarstellung eines erfindungsgemäßen Verfahrens 200 oder einer erfindungsgemäßen Anlage 200 gemäß einem zweiten Beispiel der Erfindung.

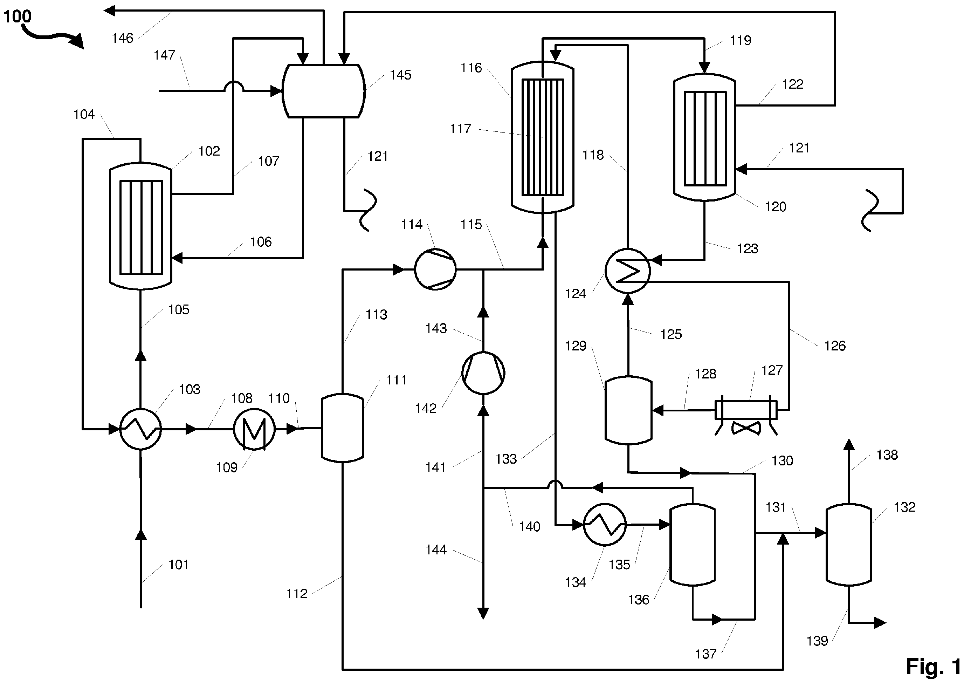

Im Beispiel gemäß Figur 1 umfasst das erfindungsgemäße Verfahren eine als Synthesekreislauf ausgestaltete Hauptreaktorstufe. Der Synthesekreislauf umfasst einen wassergekühlten Reaktor (water cooled reactor - WCR) und einen gasgekühlten Reaktor (gas cooled reactor - GCR) als Teilreaktorstufen. Über Leitung 101 wird dem Vorreaktor 102 Synthesegas aus einer Kohlevergasungsanlage bei einem Druck von 50 bar zugeführt. Die Vorreaktorstufe gemäß Figur 1 umfasst lediglich den Vorreaktor 102. Synthesegas in Leitung 101 weist einen CO-Gehalt von 29,3 Vol.-% auf, einen $H_2$-Gehalt von 67,8 Vol.-%, einen $CO_2$-Gehalt von 2,53 Vol.-% und als Restbestandteile Methan ($CH_4$) sowie inerte Bestandteile wie beispielsweise Stickstoff ($N_2$).

Synthesegas aus Leitung 101 wird in Wärmeaustauscher 103 gegen methanolhaltigen Produktstrom aus Leitung 104 vorgewärmt und über Leitung 105 in Vorreaktor 102 eingeschleust. Vorreaktor 102 ist als wassergekühlter Reaktor ausgestaltet und wird durch unter erhöhtem Druck stehendes siedendes Wasser gekühlt. Das Wasser wird über Leitung 106 aus Dampftrommel 145 zugeführt und auf der Kühlseite des Reaktors zumindest teilweise verdampft. Dampftrommel 145 wird mit Kesselspeisewasser 147 gespeist. Der Wasserdampf oder ein teilverdampfter Strom verlässt die Kühlseite des Reaktors über Leitung 107 und wird Dampftrommel 145 zugeführt. Der erzeugte Wasserdampf wird als Exportdampf 146 aus Dampftrommel 145 ausgeschleust und kann beispielsweise als Heizdampf an anderer Stelle verwendet werden.

In Vorreaktor 102 werden CO, $H_2$ sowie $CO_2$ bei einer Kühltemperatur von circa 230 °C an einem auf Kupfer basierenden Festbettkatalysator teilweise zu Methanol umgesetzt. Daraus resultierender erster methanolhaltiger Produktstrom in Leitung 104 wird in Wärmeaustauscher 103 gegen Synthesegas aus Leitung 101 vorgekühlt und über Leitung 108 Produktkühler 109 zugeführt. Gekühltes methanolhaltiges Produkt gelangt anschließend über Leitung 110 in Abscheider 111, in dem eine Trennung des methanolhaltigen Produktstroms in flüssige und gasförmige Phase stattfindet. Kondensiertes Rohmethanol wird über Leitung 112 aus Abscheider 111 abgezogen und mit Rohmethanol aus Leitung 131 zusammengeführt.

Nicht umgesetztes Synthesegas wird aus Abscheider 111 als Restgasstrom über Leitung 113 ausgeschleust und Verdichter (Restgaskompressor) 114 zugeführt, in dem das Restgas auf einen Druck von 80 bar verdichtet wird, bevor es über Leitung 115 als Restgasstrom zusammen mit Rückführgasstrom aus Leitung 143 in die Hauptreaktorstufe, welche einen gasgekühlten sowie wassergekühlten Reaktor umfasst, eingeschleust wird.

In der Hauptreaktorstufe wird Restgasstrom und Rückführgasstrom aus Leitung 115 zunächst durch eine Vielzahl innenliegender Rohre 117 des gasgekühlten Reaktors 116 geführt, in denen Restgas und Rückführgas aus Leitung 115 vorgewärmt werden, während reagierendes Restgas und Rückführgas aus Leitung 118 auf der Au-ßenseite der innenliegenden Rohre gekühlt werden. Vorgewärmtes Restgas und Rückführgas gelangen über Leitung 119 in wassergekühlten Reaktor 120 und werden dort teilweise an einem auf Kupfer basierenden Festbettkatalysator bei einer Kühltemperatur von circa 230 °C zu Methanol umgesetzt. Wassergekühlter Reaktor 120 wird durch unter erhöhtem Druck stehendes siedendes Wasser gekühlt. Das Wasser wird über Leitung 121 aus Dampftrommel 145 zugeführt. Wasserdampf oder ein teilverdampfter Strom verlässt die Kühlseite des Reaktors über Leitung 122 und wird Dampftrommel 145 zugeführt. Der erzeugte Wasserdampf wird als Exportdampf 146 aus Dampftrommel 145 ausgeschleust und kann beispielsweise als Heizdampf an anderer Stelle verwendet werden.

Durch die Reaktion des Restgases und Rückführgases in wassergekühltem Reaktor 120 wird ein zweiter methanolhaltiger (Teil-)Produktstrom erhalten, der über Leitung 123 aus wassergekühltem Reaktor 120 ausgeschleust und in Wärmeaustauscher 124 gegen Restgas/Rückführgas aus Leitung 125 vorgekühlt wird. Der vorgekühlte methanolhaltige Produktstrom gelangt anschließend über Leitung 126 in Luftkühler 127 und über Leitung 128 in Hochdruckabscheider 129, in dem eine Trennung des methanolhaltigen Produktstroms in flüssige und gasförmige Phase stattfindet. Kondensiertes Rohmethanol wird über Leitung 130 aus Hochdruckabscheider 129 abgezogen und weiter über Leitung 131 Niederdruckabscheider 132 zugeführt. Die in Hochdruckabscheider abgetrennte Gasphase wird als Restgas- und RückführgasStrom über Leitung 125 abgezogen und in Wärmeaustauscher 124 gegen methanolhaltigen Produktstrom aus Leitung 123 erwärmt und über Leitung 118 in gasgekühlten Reaktor 116 eingeschleust. In gasgekühltem Reaktor 116 findet auf der Außenseite der innenliegenden Rohre 117 an einem Festbettkatalysator auf Kupferbasis eine teilweise Umsetzung des Restgases/Rückführgases aus Leitung 118 zu einem zweiten methanolhaltigen Produkt statt, welches über Leitung 133 als zweiter methanolhaltiger (Teil-)Produktstrom abgezogen wird. Methanolhaltiges Produkt wird anschließend in Produktkühler 134 gekühlt und über Leitung 135 Hochdruckabscheider 136 zugeführt. Aus Hockdruckabscheider 136 über Leitung 137 abgezogenes Rohmethanol wird in Leitung 131 mit Rohmethanol aus Leitung 130 und Leitung 112 zusammengeführt. Aus dem gemischten Rohmethanol in Niederdruckabscheider 132 werden weitere in Rohmethanol gelöste Gasbestandteile abgetrennt, die den Niederdruckabscheider 132 über Leitung 138 verlassen. Die aus dem Niederdruckabscheider über Leitung 138 abgezogenen Gase weisen circa 30 Vol.-% Wasserstoff sowie circa 25 Vol.-% Methan auf und können beispielsweise zur Befeuerung in einem Brenner für die Dampfreformierung genutzt werden. Kondensiertes Rohmethanol wird aus Niederdruckabscheider 132 über Leitung 139 abgezogen und wird der weiteren Aufarbeitung zur Gewinnung von Reinmethanol zugeführt.

In Hochdruckabscheider 136 abgetrenntes Gas wird als Rückführgasstrom (Recycle-Gas) über Leitung 140 aus Hochdruckabscheider 136 abgezogen und über Leitung 141 Verdichter (Rückführgas- oder Recycle-Gas-Kompressor) 142 zugeführt, in dem das Rückführgas auf einen Druck von 80 bar verdichtet wird. Über Leitung 143 wird der Rückführgasstrom mit dem Restgasstrom in Leitung 115 zusammengeführt und wieder als kombinierter Strom aus Restgas und Rückführgas zuerst dem wassergekühlten Reaktor 120 und anschließend dem gasgekühlten Reaktor 116 zugeführt.

Das Verhältnis von Rückführgasstrom zu Restgasstrom, auch als Rezirkulationsrate R bezeichnet, liegt im Beispiel gemäß Figur 1 bei 1,9. Aus dem Rückführgas in Leitung 140 wird über Leitung 144 ein Purgegas (Spülgas) abgezweigt, welches neben als solches inerten Bestandteilen wie Stickstoff und bezüglich der Methanolsynthese inerten Bestandteilen wie Methan beispielsweise auch nicht reagierten Wasserstoff enthält, der anschließend beispielsweise mit Hilfe einer Wechseldruckabsorptionsvorrichtung zurückgewonnen werden kann (nicht gezeigt).

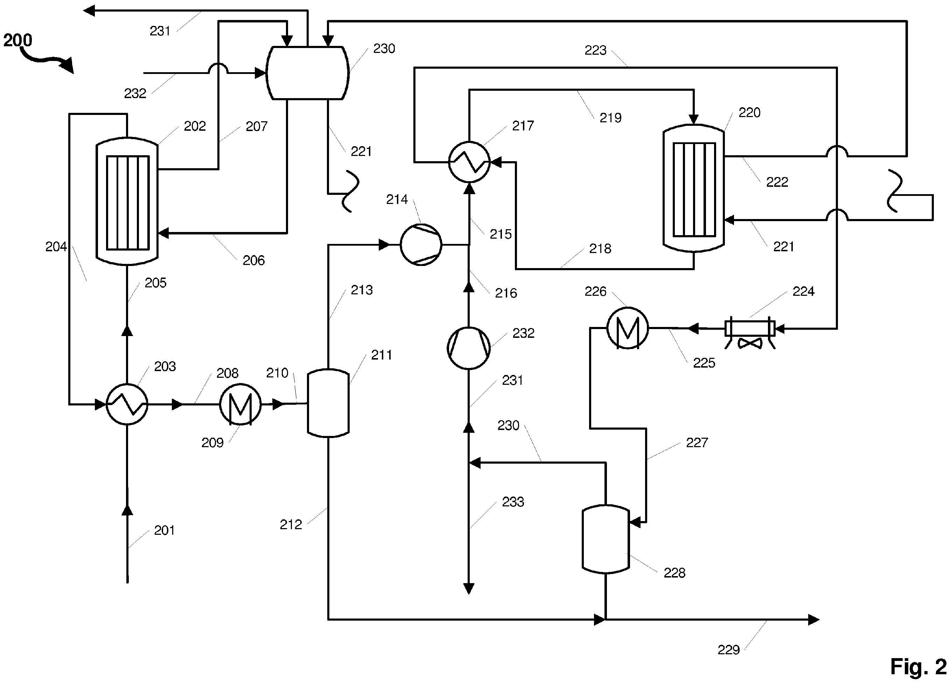

Im Beispiel gemäß Figur 2 umfasst das erfindungsgemäße Verfahren ebenfalls eine als Synthesekreislauf ausgestaltete Hauptreaktorstufe. Der Synthesekreislauf umfasst lediglich einen wassergekühlten Reaktor (water cooled reactor - WCR) als Reaktorstufe, jedoch keinen gasgekühlten Reaktor (gas cooled reactor - GCR). Über Leitung 201 wird Vorreaktor 202 Synthesegas aus einer Kohlevergasungsanlage bei einem Druck von 50 bar zugeführt. Die Vorreaktorstufe gemäß Figur 2 umfasst lediglich den Vorreaktor 202. Synthesegas in Leitung 201 weist einen CO-Gehalt von 29,3 Vol.-% auf, einen $H_2$-Gehalt von 67,8 Vol.-%, einen $CO_2$-Gehalt von 2,53 Vol.-% und als Restbestandteile Methan ($CH_4$) sowie inerte Bestandteile wie beispielsweise Stickstoff.

Synthesegas aus Leitung 201 wird in Wärmeaustauscher 203 gegen methanolhaltigen Produktstrom aus Leitung 204 vorgewärmt und über Leitung 205 in Vorreaktor 202 eingeschleust. Vorreaktor 202 ist als wassergekühlter Reaktor ausgestaltet und wird durch unter erhöhtem Druck stehendes siedendes Wasser gekühlt. Das Wasser wird über Leitung 206 aus Dampftrommel 230 zugeführt und auf der Kühlseite des Reaktors zumindest teilweise verdampft. Wasserdampf oder ein teilverdampfter Strom verlässt die Kühlseite des Reaktors über Leitung 207 und wird Dampftrommel 230 zugeführt. Der erzeugte Wasserdampf wird als Exportdampf 231 aus Dampftrommel 230 ausgeschleust und kann beispielsweise als Heizdampf an anderer Stelle verwendet werden.

In Vorreaktor 202 werden CO, $H_2$ sowie $CO_2$ bei einer Kühltemperatur von circa 230 °C an einem auf Kupfer basierenden Festbettkatalysator teilweise zu Methanol umgesetzt. Daraus resultierender erster methanolhaltiger Produktstrom in Leitung 204 wird in Wärmeaustauscher 203 gegen Synthesegas aus Leitung 201 vorgekühlt und über Leitung 208 Produktkühler 209 zugeführt. Gekühltes methanolhaltiges Produkt gelangt anschließend über Leitung 210 in Abscheider 211, in dem eine Trennung des methanolhaltigen Produktstroms in flüssige und gasförmige Phase stattfindet. Flüssiges Rohmethanol wird über Leitung 212 aus Abscheider 211 abgezogen und mit Rohmethanol aus Leitung 229 zusammengeführt.

Nicht umgesetztes Synthesegas wird aus Abscheider 211 als Restgasstrom über Leitung 213 ausgeschleust und Verdichter (Restgaskompressor) 214 zugeführt, in dem das Restgas auf einen Druck von 80 bar verdichtet wird, bevor es über Leitung 215 als Restgasstrom zusammen mit Rückführgasstrom aus Leitung 216 in die Hauptreaktorstufe, welche ausschließlich einen wassergekühlten Reaktor umfasst, eingeschleust wird.

In der Hauptreaktorstufe wird Restgasstrom und Rückführgasstrom aus Leitung 215 zunächst in Wärmeaustauscher 217 gegen zweiten methanolhaltigen Produktstrom aus Leitung 218 vorgewärmt und anschließend über Leitung 219 in den wassergekühlten Reaktor 220 eingeschleust. In wassergekühltem Reaktor 220 werden Restgas/Rückführgas teilweise an einem auf Kupfer basierenden Festbettkatalysator bei einer Kühltemperatur von circa 230 °C zu Methanol umgesetzt. Wassergekühlter Reaktor 220 wird durch unter erhöhtem Druck stehendes siedendes Wasser, welches über Leitung 221 zugeführt wird, gekühlt. Wasserdampf oder ein teilverdampfter Strom verlässt die Kühlseite des Reaktors über Leitung 222 und wird Dampftrommel 230 zugeführt. Der in Dampftrommel 230 erzeugte Wasserdampf wird als Exportdampf 231 aus Dampftrommel 230 ausgeschleust und kann beispielsweise als Heizdampf an anderer Stelle verwendet werden. Durch die Reaktion des Restgases und Rückführgases in wassergekühltem Reaktor 220 wird ein zweiter methanolhaltiger (Teil-)Produktstrom erhalten, der über Leitung 218 aus wassergekühltem Reaktor 220 ausgeschleust und in Wärmeaustauscher 217 gegen Restgas/Rückführgas aus Leitung 215 vorgekühlt wird. Der vorgekühlte methanolhaltige Produktstrom gelangt anschließend über Leitung 223 in Luftkühler 224, über Leitung 225 in Produktkühler 226 und schließlich über Leitung 227 in Abscheider 228, in dem eine Trennung des methanolhaltigen Produktstroms in flüssige und gasförmige Phase stattfindet. Rohmethanol wird über Leitung 229 aus Abscheider 228 abgezogen und kann beispielsweise zusammen mit Rohmethanol aus Leitung 212 der weiteren Aufarbeitung zu reinem Methanol unterzogen werden.

In Abscheider 228 abgetrenntes Gas wird als Rückführgasstrom (Recycle-Gas) über Leitung 230 aus Abscheider 228 abgezogen und über Leitung 231 Verdichter (Rückführgas- oder Recycle-Gas-Kompressor) 232 zugeführt, in dem das Rückführgas auf einen Druck von 80 bar verdichtet wird. Über Leitung 216 wird der Rückführgasstrom mit dem Restgasstrom in Leitung 215 zusammengeführt und als kombinierter Strom aus Restgas und Rückführgas dem wassergekühlten Reaktor 220 zugeführt.

Das Verhältnis von Rückführgasstrom zu Restgasstrom, auch als Rezirkulationsrate R bezeichnet, liegt im Beispiel gemäß Figur 2 bei 2,4. Aus dem Rückführgas in Leitung 230 wird über Leitung 233 ein Purgegas (Spülgas) abgezweigt, welches neben als solches inerten Bestandteilen wie Stickstoff auch bei der Methanolsynthese inerte Bestandteile wie Methan sowie beispielsweise nicht reagierten Wasserstoff enthält, der anschließend beispielsweise mit Hilfe einer Wechseldruckabsorptionsvorrichtung zurückgewonnen werden kann (nicht gezeigt).

Die folgenden Zahlenbeispiele sollen die technische Wirkung der vorliegenden Erfindung weiter verdeutlichen.

In der folgenden Tabelle, aufweisend drei Vergleichsbeispiele (nicht erfindungsgemäß) wurde die Wirkung des Vorreaktors in Bezug auf mögliche Einsparungen bezüglich der gesamten Kompressorleistung (Leistung des Restgas-Kompressors und Rückführgas-Kompressors) für ein Synthesegas aus combined reforming untersucht. Das Synthesegas weist einen relativ niedrigen Kohlenmonoxid-Gehalt von 20,9 Vol.-% und einen relativ hohen Kohlendioxid-Gehalt von 8,46 Vol.-% auf. Die erforderliche Kompressorleistung von 31,5 MW gemäß Vergleichsbeispiel 1 (ohne Vorreaktor) wurde auf 100 % normiert. Im Falle von Vergleichsbeispiel 2 (mit Vorreaktor, Konfiguration entspricht der Darstellung von Figur 1) wurde eine Einsparung von 5 % erzielt. Im Falle von Vergleichsbeispiel 3 (mit Vorreaktor, Konfiguration entspricht der Darstellung von Figur 2) wurde eine Einsparung von 19,5 % erzielt. Einsparungen von unter 10 % bei der Gesamtkompressorleistung rechtfertigen nicht die Verwendung eines Vorreaktors. Im Vergleichsbeispiel ist die Einsparung zwar höher, jedoch signifikant niedriger als im analogen Beispiel 2, in dem das erforderliche Gesamtkatalysatorvolumen außerdem signifikant niedriger ist.

| | Vergleichsbeispiel 1 (ohne Vorreaktor) (WCR+GCR) | Vergleichsbeispiel 2 (mit Vorreaktor) (WCR+GCR) | Vergleichsbeispiel 3 (mit Vorreaktor) (nur WCR) |
|---|---|---|---|
| Setup | Kein Reaktor bei niedrigem Prozessdruck; zwei Reaktoren bei hohem Prozessdruck | 1 Reaktor bei niedrigem Prozessdruck; zwei Reaktoren bei hohem Prozessdruck | 1 Reaktor bei niedrigem Prozessdruck; 1 Reaktor bei hohem Prozessdruck |
| CO Gehalt / Vol.-% | 20,9 | 20,9 | 20,9 |
| $H_2$ Gehalt / Vol.-% | 68,7 | 68,7 | 68,7 |
| $CO_2$ Gehalt / Vol.-% | 8,46 | 8,46 | 8,46 |
| $CH_4 + N_2$ | Rest | Rest | Rest |
| Druck Vorreaktor / bar | 30 | 30 | 30 |
| Druck WCR / bar | 76 | 76 | 76 |
| Druck GCR / bar | 74 | 74 | n/a |
| Methanol-Produktion / tons/d | 5053,1 | 5053,1 | 5053,1 |
| Kompressorleistung gesamt / MW | 31,5 | 29,9 | 25,4 |
| Rezirkulationsrate | 2,1 | 2,2 | 4,1 |
| Katalysatorvolumen / $m^3$ | 335,2 | 352,6 | 319,7 |
| Raum-Zeit-Ausbeute gesamt / kg/l/h | 0,6 | 0,6 | 0,7 |
| Kompressorleistung gesamt normiert / % | 100,0 | 95,0 | 80,5 |
| Katalysatorvolumen normiert / % | 100,0 | 105,2 | 95,4 |

In der folgenden Tabelle, aufweisend ein viertes Vergleichsbeispiel (nicht erfindungsgemäß) und zwei erfindungsgemäße Beispiele, wurde die Wirkung des Vorreaktors in Bezug auf mögliche Einsparungen bezüglich der Kompressorleistung für ein Synthesegas aus Kohlevergasung untersucht. Das Synthesegas weist im Vergleich zum Fall der obigen Tabelle einen deutlich höheren Kohlenmonoxid-Gehalt von 29,3 Vol.-% und einen deutlich niedrigeren Kohlendioxid-Gehalt von 2,53 Vol.-% auf. Die erforderliche Kompressorleistung von 17,1 MW gemäß Vergleichsbeispiel 4 (ohne Vorreaktor) wurde auf 100 % normiert. Beispiel 1 entspricht der Konfiguration wie in Figur 1 und der zugehörigen Beschreibung dargestellt. Beispiel 2 entspricht der Konfiguration wie in Figur 2 und der zugehörigen Beschreibung dargestellt. Im Falle von Beispiel 1 wurde bezüglich der Kompressorleistung gegenüber Vergleichsbeispiel 4 (ohne Vorreaktor) eine überraschend hohe prozentuale Einsparung von 31,7 % erzielt. Im Falle von Beispiel 2 wurde bezüglich der Kompressorleistung gegenüber Vergleichsbeispiel 4 (ohne Vorreaktor) eine überraschend hohe prozentuale Einsparung von 27,9 % erzielt. Es besteht somit ein synergistischer Effekt hinsichtlich möglicher Einsparungen bei der Kompressorleistung, wenn ein Vorreaktor-Konzept in Verbindung mit Einsatzgasen welche einen hohen CO-Gehalt aufweisen verfolgt wird. Mit anderen Worten, Einsatzgase mit erfindungsgemäß hohen Kohlenmonoxid-Gehalten rechtfertigen den Einsatz eines Vorreaktors aufgrund signifikanter Einsparungen bezüglich der gesamt aufzuwendenden Kompressorleistung.

Gleichzeitig verkleinert sich gemäß Beispiel 2 das erforderliche Gesamtkatalysatorvolumens bei gleichbleibender Produktionsmenge signifikant um 17,4 % und die Raum-Zeit-Ausbeute verbessert sich in vorteilhafter Weise.

|  | Vergleichsbeispiel 4 ohne Vorreaktor (WCR+GCR) | Beispiel 1 (mit Vorreaktor) (WCR+GCR) | Beispiel 2 (mit Vorreaktor) (nur WCR) |
|---|---|---|---|
| Setup | Kein Reaktor bei niedrigem Prozessdruck; zwei Reaktoren bei hohem Prozessdruck | 1 Reaktor bei niedrigem Prozessdruck; zwei Reaktoren bei hohem Prozessdruck | 1 Reaktor bei niedrigem Prozessdruck; 1 Reaktor bei hohem Prozessdruck |
| CO Gehalt / Vol.-% | 29,3 | 29,3 | 29,3 |
| $H_2$ Gehalt / Vol.-% | 67,8 | 67,8 | 67,8 |
| $CO_2$ Gehalt / Vol.-% | 2,53 | 2,53 | 2,53 |
| $CH_4 + N_2$ | Rest | Rest | Rest |
| Druck Vorreaktor / bar | 50 | 50 | 50 |
| Druck WCR / bar | 80 | 80 | 80 |
| Druck GCR / bar | 78 | 78 | n/a |
| Methanol-Produktion / tons/d | 5440,5 | 5440,5 | 5440,5 |
| Kompressorleistung gesamt / MW | 17,1 | 11,7 | 12,3 |
| Rezirkulationsrate | 1,6 | 1,9 | 2,4 |
| Katalysatorvolumen / $m^3$ | 176,8 | 186,9 | 146,0 |
| Raum-Zeit-Ausbeute gesamt / kg/l/h | 1,3 | 1,2 | 1,6 |
| Kompressorleistung gesamt normiert / % | 100,0 | **68,3** | **72,1** |
| Katalysatorvolumen normiert / % | 100,0 | 105,7 | **82,6** |

Ausführungsformen der Erfindung werden unter Bezugnahme auf verschiedene Arten von Gegenständen beschrieben. Insbesondere werden bestimmte Ausführungsformen unter Bezugnahme auf Ansprüche des Verfahrenstyps beschrieben, während andere Ausführungsformen unter Bezugnahme auf die Ansprüche des Vorrichtungstyps beschrieben werden. Ein Fachmann wird jedoch aus der obigen und der folgenden Beschreibung entnehmen, dass, außer anders angegeben, zusätzlich zu irgendeiner Kombination von Merkmalen, die zu einer Art von Anspruchstyp gehören, auch jede Kombination von Merkmalen in Bezug auf verschiedene Arten von Gegenständen oder Anspruchstypen in Betracht gezogen werden kann. Alle Merkmale können kombiniert werden um synergetische Effekte zu erzielen, welche über die einfache Summierung der technischen Merkmale hinausgehen.

Während die Erfindung im Detail in der Zeichnung und der vorhergehenden Beschreibung dargestellt und beschrieben wurde, sollen eine solche Darstellung und Beschreibung als veranschaulichend oder beispielhaft und nicht einschränkend betrachtet werden. Die Erfindung ist nicht auf die offenbarten Ausführungsformen beschränkt, sondern lediglich durch die Ansprüche.

In den Ansprüchen schließt das Wort "aufweisend" oder "umfassend" weitere Elemente oder Schritte nicht aus, und der unbestimmte Artikel "ein" oder "eine" schließt eine Mehrzahl nicht aus.

**Bezugszeichenliste**

| 100 | Verfahren oder Anlage |
|---|---|
| 101 | Leitung |
| 102 | Vorreaktor |
| 103 | Wärmeaustauscher |
| 104 - 108 | Leitung |
| 109 | Produktkühler |
| 110 | Leitung |
| 111 | Abscheider |
| 112, 113 | Leitung |
| 114 | Verdichter |
| 115 | Leitung |
| 116 | Gasgekühlter Reaktor (GCR) |
| 117 | Innenliegende Rohre |
| 118, 119 | Leitung |
| 120 | Wassergekühlter Reaktor (WCR) |
| 121 - 123 | Leitung |
| 124 | Wärmeaustauscher |
| 125, 126 | Leitung |
| 127 | Luftkühler |
| 128 | Leitung |
| 129 | Hochdruckabscheider |
| 130, 131 | Leitung |
| 132 | Niederdruckabscheider |
| 133 | Leitung |
| 134 | Produktkühler |
| 135 | Leitung |
| 136 | Hochdruckabscheider |
| 137 - 141 | Leitung |
| 142 | Verdichter |
| 143, 144 | Leitung |
| 145 | Dampftrommel |
| 146 | Exportdampf |
| 147 | Kesselspeisewasser |

| 200 | Verfahren oder Anlage |
|---|---|
| 201 | Leitung |
| 202 | Vorreaktor |
| 203 | Wärmeaustauscher |
| 204 - 208 | Leitung |
| 209 | Produktkühler |
| 210 | Leitung |
| 211 | Abscheider |
| 212, 213 | Leitung |
| 214 | Verdichter |
| 215, 216 | Leitung |
| 217 | Wärmeaustauscher |
| 218, 219 | Leitung |
| 220 | Wassergekühlter Reaktor |
| 221 - 223 | Leitung |
| 224 | Luftkühler |
| 225 | Leitung |
| 226 | Produktkühler |
| 227 | Leitung |
| 228 | Abscheider |
| 229 | Leitung |
| 230 | Dampftrommel |
| 231 | Exportdampf |
| 232 | Kesselspeisewasser |

**Patentansprüche**

1. Verfahren zur Herstellung von Methanol aus einem Kohlenmonoxid (CO) und Wasserstoff ($H_2$) aufweisenden Einsatzgas, bei dem

   unter erhöhtem Druck erzeugtes und stehendes Einsatzgas als Einsatzgasstrom zur katalytischen Umsetzung in einen ersten methanolhaltigen Produktstrom in eine Vorreaktorstufe eingeschleust wird, und
   Methanol aus dem ersten methanolhaltigen Produktstrom abgetrennt und aus der Vorreaktorstufe ausgeschleust wird, und
   Restgas des verbleibenden Einsatzgasstroms als Restgasstrom auf Reaktionsdruck verdichtet und zur katalytischen Umsetzung in einen zweiten methanolhaltigen Produktstrom in eine Hauptreaktorstufe eingeschleust wird, und Methanol aus dem zweiten methanolhaltigen Produktstrom abgetrennt und aus der Hauptreaktorstufe ausgeschleust wird,
   **dadurch gekennzeichnet, dass**
   das Einsatzgas vor dem Einschleusen in die Vorreaktorstufe einen Kohlenmonoxid-Gehalt von 25 Vol.-% bis 36 Vol.-% aufweist.

2. Verfahren nach Anspruch 1, **dadurch gekennzeichnet, dass** das Einsatzgas vor dem Einschleusen in die Vorreaktorstufe einen Wasserstoff-Gehalt von 66 Vol.-% bis 72 Vol.-% aufweist.

3. Verfahren nach Anspruch 1 oder 2, **dadurch gekennzeichnet, dass** das Einsatzgas vor dem Einschleusen in die Vorreaktorstufe einen Kohlendioxid-Gehalt ($CO_2$ - Gehalt) von kleiner oder gleich 5 Vol.-% aufweist.

4. Verfahren nach einem der vorherigen Ansprüche, **dadurch gekennzeichnet, dass** das Einsatzgas vor dem Einschleusen in die Vorreaktorstufe eine Stöchiometriezahl SN, wobei gilt

$$SN = \frac{n(H_2) - n(CO_2)}{n(CO) + n(CO_2)} \, ,$$

   *mit n in [mol]*,
   von 1,5 bis 3,0 aufweist.

5. Verfahren nach einem der vorherigen Ansprüche, **dadurch gekennzeichnet, dass** das Einsatzgas einen Druck von 20 bis 100 bar aufweist, vorzugsweise einen Druck von 35 bis 55 bar aufweist.

6. Verfahren nach einem der vorherigen Ansprüche, **dadurch gekennzeichnet, dass** das unter erhöhtem Druck erzeugte und stehende Einsatzgas ohne weitere Druckerhöhung in die Vorreaktorstufe eingeschleust wird.

7. Verfahren nach einem der vorherigen Ansprüche, **dadurch gekennzeichnet, dass** das Restgas des Restgasstroms vor dem Einschleusen in die Hauptreaktorstufe auf einen gegenüber dem Einsatzgas höheren Druck verdichtet wird.

8. Verfahren nach einem der vorherigen Ansprüche, **dadurch gekennzeichnet, dass** die Hauptreaktorstufe Teil eines Synthesekreislaufs ist, wobei ein nach Abtrennung des Methanols aus dem zweiten methanolhaltigen Produktstrom verbleibender Rückführgasstrom zur Hauptreaktorstufe im Synthesekreislauf zurückgeführt und mit dem Restgasstrom zusammengeführt wird.

9. Verfahren nach Anspruch 8, **dadurch gekennzeichnet, dass** für eine Rezirkulationsrate R, definiert als

$$R = \frac{Volumenstrom \ (Rückführgasstrom)}{Volumenstrom \ (Restgasstrom)} \, ,$$

   $R \leq 2,5$ gilt.

10. Verfahren nach einem der Ansprüche 1 bis 7, **dadurch gekennzeichnet, dass** die Hauptreaktorstufe eine Anzahl n seriell angeordneter Teilreaktorstufen umfasst, und der zweite methanolhaltige Produktstrom n methanolhaltige Teilproduktströme umfasst, wobei Methanol aus den n methanolhaltigen Teilproduktströmen abgetrennt und aus

der Hauptreaktorstufe ausgeschleust wird.

**11.** Verfahren nach einem der vorherigen Ansprüche, **dadurch gekennzeichnet, dass** die Vorreaktorstufe einen wassergekühlten Reaktor umfasst.

**12.** Verfahren nach Anspruch 11, **dadurch gekennzeichnet, dass** der wassergekühlte Reaktor eine Kühltemperatur von 180 °C bis 250 °C aufweist.

**13.** Verfahren nach einem der vorherigen Ansprüche, **dadurch gekennzeichnet, dass** die Hauptreaktorstufe einen wassergekühlten Reaktor umfasst.

**14.** Verfahren nach Anspruch 13, **dadurch gekennzeichnet, dass** die Hauptreaktorstufe zusätzlich einen gasgekühlten Reaktor umfasst.

**15.** Verfahren nach Anspruch 13, **dadurch gekennzeichnet, dass** die Hauptreaktorstufe keinen gasgekühlten Reaktor umfasst.

**16.** Verwendung einer Anlage zur Herstellung von Methanol aus einem Kohlenmonoxid (CO) und Wasserstoff ($H_2$) aufweisenden Einsatzgas in einem Verfahren nach einem der Ansprüche 1 bis 15 zur Umsetzung eines Einsatzgases mit einem Kohlenmonoxid-Gehalt von 25 Vol.-% bis 36 Vol.-%.

**17.** Verwendung nach Anspruch 16, wobei das Einsatzgas mittels Vergasung von Kohle, autothermes Reformieren oder Gas-POX erzeugt wurde.

**Claims**

**1.** Process for producing methanol from an input gas comprising carbon monoxide (CO) and hydrogen ($H_2$), wherein

input gas produced at and under elevated pressure is introduced into a pre-reactor stage as an input gas stream for catalytic conversion into a first methanol-containing product stream and
methanol is separated from the first methanol-containing product stream and discharged from the pre-reactor stage and
residual gas of the remaining input gas stream is compressed to reaction pressure as a residual gas stream and introduced into a main reactor stage for catalytic conversion into a second methanol-containing product stream and
methanol is separated from the second methanol-containing product stream and discharged from the main reactor stage,
**characterized in that**
the input gas has a carbon monoxide content of 25% to 36% by volume before introduction into the pre-reactor stage.

**2.** Process according to Claim 1, **characterized in that** the input gas has a hydrogen content of 66% to 72% by volume before introduction into the pre-reactor stage.

**3.** Process according to Claim 1 or 2, **characterized in that** the input gas has a carbon dioxide content ($CO_2$ content) of not more than 5% by volume before introduction into the pre-reactor stage.

**4.** Process according to any of the preceding claims, **characterized in that** the input gas has a stoichiometry number SN of 1.5 to 3.0 before introduction into the pre-reactor stage, wherein

$$SN = \frac{n(H_2) - n(CO_2)}{n(CO) + n(CO_2)}$$

, where n is in [mol].

**5.** Process according to any of the preceding claims, **characterized in that** the input gas has a pressure of 20 to 100 bar, preferably a pressure of 35 to 55 bar.

6. Process according to any of the preceding claims, **characterized in that** the input gas produced and present under elevated pressure is introduced into the pre-reactor stage without further pressurization.

7. Process according to any of the preceding claims, **characterized in that** the residual gas of the residual gas stream is compressed to a pressure higher than that of the input gas before introduction into the main reactor stage.

8. Process according to any of the preceding claims, **characterized in that** the main reactor stage is part of a synthesis circuit, wherein a recycle gas stream remaining after separation of the methanol from the second methanol-containing product stream is recycled to the main reactor stage in the synthesis circuit and combined with the residual gas stream.

9. Process according to Claim 8, **characterized in that** for a recirculation rate R defined as

$$R = \frac{volume\ flow\ (recycle\ gas\ stream)}{volume\ flow\ (residual\ gas\ stream)},$$

R ≤ 2.5.

10. Process according to any of Claims 1 to 7, **characterized in that** the main reactor stage comprises a number n of serially arranged partial reactor stages and the second methanol-containing product stream n comprises methanol-containing partial product streams, wherein methanol is separated from the n methanol-containing partial product streams and discharged from the main reactor stage.

11. Process according to any of the preceding claims, **characterized in that** the pre-reactor stage comprises a water-cooled reactor.

12. Process according to Claim 11, **characterized in that** the water-cooled reactor has a cooling temperature of 180°C to 250°C.

13. Process according to any of the preceding claims, **characterized in that** the main reactor stage comprises a water-cooled reactor.

14. Process according to Claim 13, **characterized in that** the main reactor stage additionally comprises a gas-cooled reactor.

15. Process according to Claim 13, **characterized in that** the main reactor stage does not comprise a gas-cooled reactor.

16. Use of a plant for producing methanol from an input gas comprising carbon monoxide (CO) and hydrogen ($H_2$) in a process according to any of claims 1 to 15 for converting an input gas having a carbon monoxide content of 25% to 36% by volume.

17. Use according to Claim 16, wherein the input gas has been produced by gasification of coal, autothermal reforming or gas POX.


**Revendications**

1. Procédé pour la préparation de méthanol à partir d'un gaz d'alimentation comprenant du monoxyde de carbone (CO) et de l'hydrogène ($H_2$), dans lequel

du gaz d'alimentation produit et maintenu sous pression élevée est introduit comme flux de gaz d'alimentation pour une transformation catalytique dans un premier flux de produit contenant du méthanol dans un étage de préréacteur, et
du méthanol est séparé du premier flux de produit contenant du méthanol et est évacué de l'étage de préréacteur, et
du gaz résiduel du flux de gaz d'alimentation restant est condensé à la pression de réaction en tant que flux de gaz résiduel et introduit pour une transformation catalytique dans un deuxième flux de produit contenant du méthanol dans un étage de réacteur principal, et

du méthanol est séparé du deuxième flux de produit contenant du méthanol et évacué de l'étage de réacteur principal,

**caractérisé en ce que**

le gaz d'alimentation présente, avant l'introduction dans l'étage de préréacteur, une teneur en monoxyde de carbone de 25 % en volume à 36 % en volume.

2. Procédé selon la revendication 1, **caractérisé en ce que** le gaz d'alimentation présente, avant l'introduction dans l'étage de préréacteur, une teneur en hydrogène de 66 % en volume à 72 % en volume.

3. Procédé selon la revendication 1 ou 2, **caractérisé en ce que** le gaz d'alimentation, avant l'introduction dans l'étage de préréacteur, présente une teneur en dioxyde de carbone (teneur en $CO_2$) inférieure ou égale à 5 % en volume.

4. Procédé selon l'une quelconque des revendications précédentes, **caractérisé en ce que** le gaz d'alimentation, avant l'introduction dans le préréacteur, présente un indice de stoechiométrie SN de 1,5 à 3,0, avec

$$SN = \frac{n(H_2) - n(CO_2)}{n(CO) + n(CO_2)},$$

avec n en [mol].

5. Procédé selon l'une quelconque des revendications précédentes, **caractérisé en ce que** le gaz d'alimentation présente une pression de 20 à 100 bars, de préférence une pression de 35 à 55 bars.

6. Procédé selon l'une quelconque des revendications précédentes, **caractérisé en ce que** le gaz d'alimentation produit et maintenu sous pression élevée est introduit dans l'étage de préréacteur sans élévation de pression supplémentaire.

7. Procédé selon l'une quelconque des revendications précédentes, **caractérisé en ce que** le gaz résiduel du flux de gaz résiduel, avant l'introduction dans l'étage de réacteur principal, est condensé à une pression supérieure à celle du gaz d'alimentation.

8. Procédé selon l'une quelconque des revendications précédentes, **caractérisé en ce que** l'étage de réacteur principal fait partie d'un circuit de synthèse, un flux de gaz de recyclage restant après la séparation du méthanol à partir du deuxième flux de produit contenant du méthanol étant recyclé à l'étage de réacteur principal dans le circuit de synthèse et réuni avec le flux de gaz résiduel.

9. Procédé selon la revendication 8, **caractérisé en ce que** pour une vitesse de recirculation R, définie comme

```
R = flux volumique (flux de gaz de recyclage)/flux
        volumique (flux de gaz résiduel),
```

alors R ≤ 2,5.

10. Procédé selon l'une quelconque des revendications 1 à 7, **caractérisé en ce que** l'étage de réacteur principal comprend un nombre n d'étages partiels de réacteur agencés en série, et le deuxième flux de produit contenant du méthanol comprend n flux partiels de produit contenant du méthanol, dans lequel du méthanol est séparé des n flux partiels de produit contenant du méthanol et évacué de l'étage de réacteur principal.

11. Procédé selon l'une quelconque des revendications précédentes, **caractérisé en ce que** l'étage de préréacteur comprend un réacteur refroidi à l'eau.

12. Procédé selon la revendication 11, **caractérisé en ce que** le réacteur refroidi à l'eau présente une température de refroidissement de 180 °C à 250 °C.

**13.** Procédé selon l'une quelconque des revendications précédentes, **caractérisé en ce que** l'étage de réacteur principal comprend un réacteur refroidi à l'eau.

**14.** Procédé selon la revendication 13, **caractérisé en ce que** l'étage de réacteur principal comprend de plus un réacteur refroidi au gaz.

**15.** Procédé selon la revendication 13, **caractérisé en ce que** l'étage de réacteur principal ne comprend pas de réacteur refroidi au gaz.

**16.** Utilisation d'une installation de préparation de méthanol à partir d'un gaz d'alimentation comprenant du monoxyde de carbone (CO) et de l'hydrogène ($H_2$) dans un procédé selon l'une quelconque des revendications 1 à 15 pour la transformation d'un gaz d'alimentation comportant une teneur en monoxyde de carbone de 25 % en volume à 36 % en volume.

**17.** Utilisation selon la revendication 16, le gaz d'alimentation ayant été produit au moyen d'une gazéification de charbon, d'un reformage autothermique ou de POX de gaz.

**Fig. 1**

100

Fig. 2

EP 3 741 738 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10126719 A1
- GB 2550993 A
- WO 2017121981 A